# EUROPEAN PATENT SPECIFICATION

(11) **EP 3 012 207 B1**
(45) Date of publication and mention of the grant of the patent: **25.07.2018**
(21) Application number: 14189687.8
(22) Date of filing: 21.10.2014
(51) Int. Cl.: B65D 85/07

(54) **Flexible package**
Flexible Verpackung
Emballage flexible

(43) Date of publication of application: 27.04.2016
(73) Proprietor: The Procter & Gamble Company, Cincinnati, OH 45202 (US)
(72) Inventor: Motsch, Andreas Peter, 65824 Schwalbach am Taunus (DE); Blum, Thorsten, 65824 Schwalbach am Taunus (DE)
(74) Representative: Chandrani, Vandita

(56) References cited:
- EP-A1- 2 110 337
- WO-A1-91/08962
- WO-A1-2014/018329
- US-A- 5 829 230
- US-A1- 2012 325 716

## Description

### FIELD OF THE INVENTION

A flexible package capable of receiving and holding a multiplicity of compressed articles, such as disposable absorbent articles is provided. More particularly, the invention provides a flexible package having a substantially parallelepiped-shape. A flexible package having a substantially folded flat-shape able to be converted into the unfolded flexible package having a substantially parallelepiped-shape is provided. Methods for making such flexible packages are provided.

### BACKGROUND OF THE INVENTION

Flexible packages are composed of flexible material such as a thermoplastic film. Their use for packaging products such as disposable absorbent articles, e.g. diapers, as well as feminine hygiene products, e.g. sanitary napkins is well known in the art, see for example International Patent Application WO2014/018329 A1.

US2012/325716 relates to a package which includes a plurality of disposable absorbent articles and a packaging having a front panel, a back panel, a top panel, a bottom panel, and a pair of side panels. At least one of the side panels has a longitudinal central axis, a pair of side edges, a gusset disposed adjacent the top panel, and a line of weakness having a first portion disposed on one side of the longitudinal central axis, a second portion disposed on the opposite side of the longitudinal central axis, and a third portion spanning between and connecting the first and second portions. The third portion of the line of weakness is spaced from the ends of the first and second portions. The plurality of disposable absorbent articles is compressed within the interior compartment and arranged in at least one row extending between the side panels.

The flexible package generally comprises front and rear walls which are connected to each other (e.g. sealed) along first and second opposite side walls with first and second side seams. The flexible package also includes a third and fourth opposite side walls. The third side wall may form the top wall of the flexible package. The third side wall comprises a pair of gussets. The flexible package may also include a handle to facilitate the carrying of the package. The products may be removed from a top opening.

The flexible package can be filled with a multiplicity of compressed articles, typically by entering the compressed articles from the bottom of the flexible package. For this step, a folded flat-shape package is opened and unfolded from the bottom by an appropriate device. Then, the compressed articles are introduced into the flexible package. The appropriate device in combination with the compressed articles may exert some tension on each of the first and second opposite side walls at the respective gusset, particularly along the first and second side seams. It might occur that the first and second side seams tear open due to the tension at the first and second opposite side walls. It is generally known in the industry to insert a slit at each gusset to relieve the first and second side walls from the tension exerted on them.

However, applying a slit at each gusset forms an opening in the final package filled with the compressed articles. The compressed articles are in contact with the surrounding environment and might be contaminated with dust, lint or moisture for instance.

There is hence a need to provide a flexible package able to relieve the tension exerted at the first and second side walls while keeping the compressed articles safe from any kind of contamination during at least the shelf life of the articles.

### SUMMARY OF THE INVENTION

A flexible package for accommodating a multiplicity of compressed articles as defined in claim 1.

### BRIEF DESCRIPTION OF THE DRAWINGS

While the specification concludes with claims particularly pointing out and distinctly claiming the present invention, it is believed that the same will be better understood from the following description read in conjunction with the accompanying drawings in which:
Fig. 1 is a schematic perspective view of a flexible package within the scope of the invention;
Fig. 2 is a top view of the first, second and third panels of the film in a flat state, i.e. before any folding has been performed to form the flexible package;
Fig. 3 is a side view of the flexible package taken to the inside the package to illustrate where the tension is applied on an internal panel of a gusset of the flexible package;
Fig. 4 is a side view of the flexible package of Fig. 1 illustrating a first side wall and a gusset of the flexible package;
Figs. 5A to 5H show examples of flexible packaging that is outside the scope of the present invention. For example, Fig. 5A is a portion of the side view of the flexible package of Fig. 1 comprising a line of weakness at a gusset where the thickness of the film has been reduced or the film has been perforated;
Fig. 5B is a portion of the side view of the flexible package of Fig. 1 comprising a wavy line of weakness at a gusset;
Fig. 5C is a portion of the side view of the flexible package of Fig. 1 comprising a zigzag as an area of weakness at a gusset;
Fig. 5D is a portion of the side view of the flexible package of Fig. 1 comprising a circle as an area of weakness at a gusset;
Fig. 5E is a portion of the side view of the flexible package of Fig. 1 comprising a straight line of weakness at a gusset;
Fig. 5F is a portion of the side view of the flexible package of Fig. 1 comprising a straight line of weakness at a gusset;
Fig. 5G is a portion of the side view of the flexible package of Fig. 1 comprising a straight line of weakness at a gusset;
Fig. 5H is a portion of the side view of the flexible package of Fig. 1 comprising a straight line of weakness at a gusset;
Fig. 5I shows an example of the present invention with a portion of the side view of the flexible package of Fig. 1 comprising a straight line of weakness comprising a first and second portion of weakness at a gusset;
Fig. 6 is a front view of the flexible package having a substantially folded flat-shape; Fig. 7 is a front view of flexible packages during manufacturing from an endless sheet of a film;
Fig. 8 is a cross-sectional view of the endless sheet of the film of Fig. 7 along a cutting plane 8-8;
Fig. 9 is a front view of flexible packages during manufacturing from an endless sheet of a film after formation of a plurality of areas of weakness;
Fig. 10 is in a front view of two flexible packages during manufacturing after formation of the side seams;
Fig. 11 is a top view from an endless sheet of a film after formation of a plurality of areas of weakness.

### DETAILED DESCRIPTION OF THE INVENTION

### Definition of terms

The term "absorbent article" as used herein refers to devices which absorb and contain body exudates, and, more specifically, refers to devices which are placed against or in proximity to the body of the wearer to absorb and contain the various exudates discharged from the body. Typical absorbent articles of the present invention include but are not limited to diapers, adult incontinence briefs, training pants, diaper holders and liners, absorbent inserts and the like, as well as feminine hygiene products, such as sanitary napkins and panty liners, and the like. Absorbent articles also include wipes, such as household cleaning wipes, baby wipes, and the like.

The term "altitude" as used herein refers to a line segment in a triangle extending from a vertex of the triangle towards an opposite side of the vertex and forms a right angle with the opposite side. For instance, the altitude of each internal panel of each gusset extends from the vertex of each internal panel of each gusset towards the third side wall. The altitude of the internal panel of each gusset forms a right angle with the third side wall.

"Comprise", "comprising", and "comprises" as used herein are open ended terms, each specifying the presence of what follows, e.g., a component, but not precluding the presence of other features, e.g., elements, steps or components known in the art, or disclosed herein.

The term "compressed article" as used herein means that the articles have been compressed in a flexible package at an In Bag Stack Height (i.e. the total caliper of 10 bi-folded or tri-folded diapers, measured while under compression) of less than 90 mm.

The term "cross machine direction" or "CD" as used herein means the direction perpendicular to the machine direction in the same plane of the endless sheet of flexible material comprising the fibrous structure.

The term "diaper" as used herein refers to absorbent articles generally worn by infants and incontinent persons about the lower torso. The diaper may be fastened onto the wearer using tapes or, alternatively, the diaper may have side seams, which are fastened together - both permanently or refastenably- such that the diaper is applied onto the wear like a conventional underwear (i.e. the user will put his legs through the respective leg openings and the diaper is then pulled up to its final position).

The term "disposable" as used herein refers to absorbent articles which generally are not intended to be laundered or otherwise restored or reused (i.e., they are intended to be discarded after a single use and, preferably, to be recycled, composted or otherwise discarded in an environmentally compatible manner).

The term "film" as used herein refers to a substantially non-fibrous sheet-like material wherein the length and width of the material far exceed the thickness of the material. Typically, films have a thickness of about 0.5 mm or less.

The term "machine direction" or "MD" as used herein means the direction parallel to the flow of the endless sheet of flexible material through the flexible package making machine and/or product manufacturing equipment.

The term "vertex" as used herein refers to the end point where the first and second folding lines of each gusset intersect. The vertex of each gusset is joined to the respective first or second side seam.

As used herein the expressions "front", "rear", "outer", "inner", "longitudinal", "transversal", "top", "side", "bottom" and the like, when used to describe the flexible package, relate to a filled package placed in a carrying position with the bottom part facing downwards and the top part facing upwards, such as e.g. shown in the figures. Some of these terms are also used with respect to the methods of manufacturing of the flexible package.

### The flexible package in a parallelepiped shape

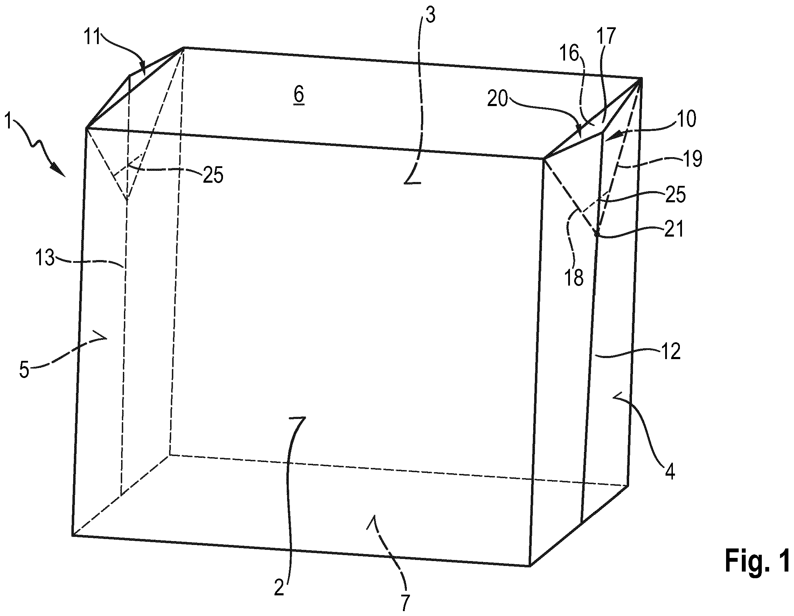

Fig. 1 shows a flexible package 1 within the scope of the invention. The flexible package 1 has a substantially parallelepiped-shape, i.e. a hexahedron shape.

The flexible package 1 may be made of a material(s) which are flexible such that the flexible package 1 may assume the parallelepiped-shape when it is filled with compressed articles.

The flexible package 1 of the present invention may comprise a thermoplastic film or a laminate of more than one thermoplastic films bonded to each other prior to making the flexible package 1. Examples of suitable materials are polyethylene, polypropylene and polyethylene terephthalate.

The flexible package 1 may comprise a multiplicity of compressed articles, such as from 10 to 200 compressed articles or from 10 to 100 compressed articles, e.g. compressed disposable absorbent articles. For example, the flexible package 1 of the present invention may be used for accommodating a multiplicity of diapers. The diapers may be folded and the multiplicity of folded diapers may be arranged in one or more rows within the package.

A diaper typically has a longitudinal axis and a transversal axis. One end portion of the diaper is configured as a front waist region of the diaper. The opposite end portion is configured as a back waist region of the diaper. An intermediate portion of the diaper is configured as a crotch region, which extends longitudinally between the front and back waist regions. The crotch region is that portion of the diaper which, when the diaper is worn, is generally positioned between the wearer's legs. Further, the transversal axis is typically in the crotch region of the diaper.

In a two-folded diaper, the diaper is folded in the crotch region along its transversal axis or near its transversal axis. In the folded diaper, the front waist region is lying adjacent the back waist region and the front and back waist region are substantially coextensive.

Alternatively, the diaper may be tri-folded, whereby one of the front or back waist region is folded over onto the crotch region where after the remaining waist region is folded over onto the first folded waist region

The flexible package 1 comprises a front wall 2, a rear wall 3 opposite to the front wall 2, a first and second opposite side wall 4, 5, a third and fourth opposite side wall 6, 7 and a pair of gussets 10, 11.

The third side wall 6 may be a top wall of the flexible package 1. The fourth side wall 7 may be a bottom wall of the flexible package 1, as shown in Fig. 1.

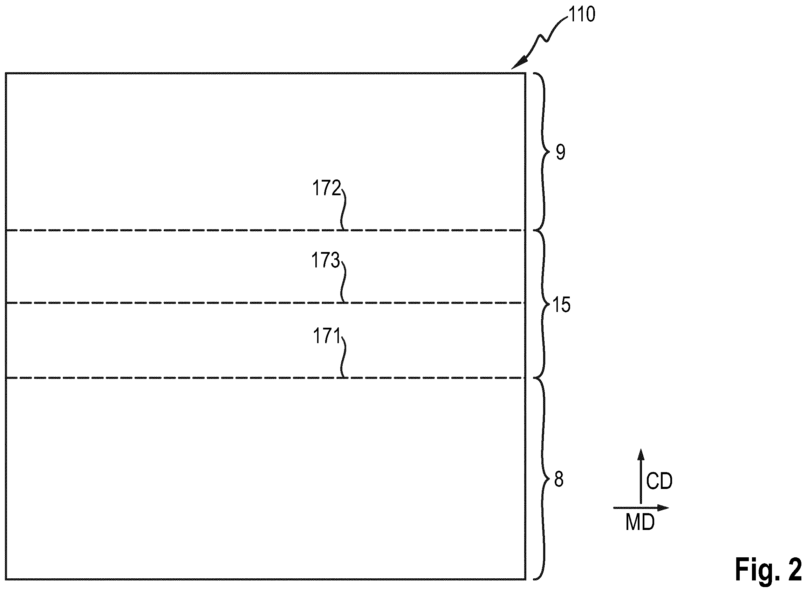

Fig. 2 shows a top view of a film 110 in a flat state, before any folding has been performed to manufacture and form the flexible package 1 from the film 110. The flexible package 1 comprises a first panel 8. The first panel 8 forms the front wall 2, a portion of each of the first and second opposite side wall 4, 5 and a portion of the fourth side wall 7. A second panel 9 forms the rear wall 3, a portion 11 of each of the first and second opposite side wall 4, 5 and a portion of the fourth side wall 7. As shown in Fig. 1, the first and the second panels 8, 9 are connected to each other at the first and second opposite side wall 4, 5 along first and second side seams 12, 13. The first and second side seams 12, 13 extend from the third side wall 6 of the package 1 to the fourth side wall 7 of the flexible package 1.

Furthermore, a third panel 15 forms the third side wall 6. The third panel 15 comprises the pair of gussets 10, 11. Each gusset 10, 11 is located at the respective first or second side wall 4, 5.

Each gusset 10 comprises an internal panel 16 and an external panel 17. The internal panel 16 and external panel 17 of each gusset 10 are made of triangular portions of the third panel 15. The internal panel 16 and external panel 17 of each gusset 10 are coextensive at a first and second fold line 18, 19 to form a pocket 20, as exemplary indicated in Fig. 1.

The first and second fold line 18, 19 of the pocket 20 of each gusset 10 converge and meet at a vertex 21. The vertex 21 of each gusset 10 is joined to the respective first or second side seam 12, 13 at the first or second side wall 4, 5.

When the flexible package 1 is filled with a multiplicity of compressed articles, e.g. by entering the compressed articles from the not yet folded and closed fourth side wall 7 of the flexible package 1, the appropriate device used to introduced the compressed articles inside the flexible package 120 together with the compressed articles may exert some tension on each of the first and second opposite side wall 4, 5 of the flexible package 1. The compressed articles may slightly expand after they have been introduced in the flexible package 1 and thus exert some tension at the first and second opposite side walls 4, 5. The tension are exerted on each of the gusset 10, 11 at the respective first and second opposite side walls 4, 5, particularly along the first and second side seams 12, 13.

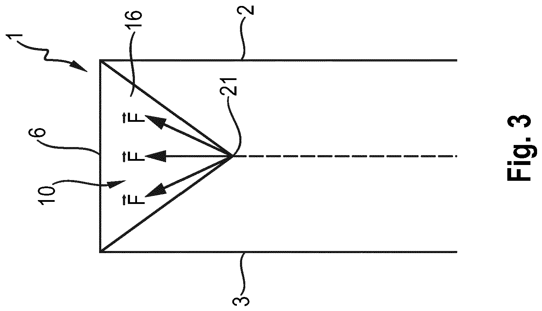

Fig. 3 is a side view of the flexible package 1 taken to the inside the package 1. Tension exerted by the compressed articles and/or by the apparatus may come from the vertex 21 of each gusset and extend from the vertex 21 of each gusset 10, 11 towards the third side wall 6. The highest tension are exerted from the vertex 21 of each gusset 10, 21 along the respective side seams 12, 13 towards the third side wall 6. It might occur that the first and second side seams 12, 13 tear open at each gusset 10, 11 due to the tension exerted at the first and second opposite side walls 4, 5.

It is known in the industry to insert a slit at each gusset 10, 11 to relieve the first and second side walls 4, 5 from the tension exerted on them. However, applying a slit at each gusset 10, 11 forms an opening. The compressed articles are in contact with the surrounding environment and might be contaminated with dust, lint or moisture for instance. Also, some consumers may find it unpleasant to have the compressed articles in contact with the surrounding environment or attempt to touch the articles by passing a finger through the opening obtained from a continuous slit in the pocket 20 of each gusset 10, 11.

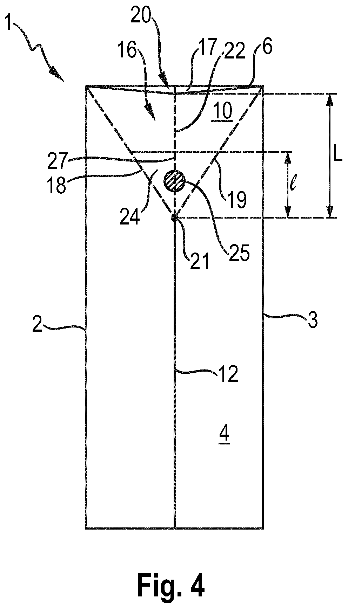

Hence, rather than providing a slit, an area of weakness 25 has been introduced at a specific location in at least one gusset 10, 11. Fig. 4 is a side view of the flexible package of Fig. 1 illustrating the first side wall 4 with the gusset 10.

The internal panel 16 of each gusset 10 comprises an altitude 22 extending from the vertex 21 of each gusset 10 towards the third side wall 6. The altitude 22 of the internal panel 16 of each gusset 10 has a length L.

The internal panel 16 of each gusset 10 comprises a triangular area 24 which starts from the vertex 21 of each gusset 10 towards the third side wall 6. The triangular area 24 of the internal panel 16 of each gusset 10 has an altitude 27 which coincides with the altitude 22 of the internal panel 16 of each gusset.

The altitude 27 of the triangular area 24 of the internal panel 16 of each gusset 10 has a length 1 which is from 1% to 30% or from 1% to 15% or from 1% to 10% or from 1% to 5% of the length L of the altitude 22 of the internal panel 16 of each gusset 10.

At least one gusset 10 comprises an area of weakness 25 located at least in the internal panel 16 of the at least one gusset 10 within the triangular area 24 of the internal panel 16 of the at least one gusset 10. The area of weakness 25 is located at or adjacent to the altitude 22 of the internal panel 16 of the at least one gusset 10.

While it is not yet fully understood, it has been found that more tension can be exerted on one of the first side wall 4 rather than the opposite second side wall 5 when the compressed articles have different sizes and/or arranged in a specific order.

Hence, it is beneficial to have at least one gusset 10 which comprises an area of weakness 25 located at least in the internal panel 16 of the at least one gusset 10 within the triangular area 24 of the internal panel 16 of the at least one gusset 10.

The area of weakness 25 is located at or adjacent to the altitude 22 of the internal panel 16 of the at least one gusset 10 because the tension is exerted from the vertex 21 of each gusset 10, 11 towards the third side wall 6, and particularly along the respective first or second side seams 12, 13. The first and second side seams 12, 13 are respectively substantially parallel to or even coincide with the altitude 22 of each internal panel 16 of each gusset 10, 11.

Each gusset 10, 11 may comprise an area of weakness 25 which is located at least within the triangular area 24 of the internal panel 16 of each gusset 10, 11. The area of weakness 25 may be located at or adjacent to the altitude 22 of the internal panel 16 of each gusset 10, 11. In that case, the flexible package may comprise substantially identical compressed absorbent articles that exert tension on each gusset at each first and second opposite side wall 4, 5.

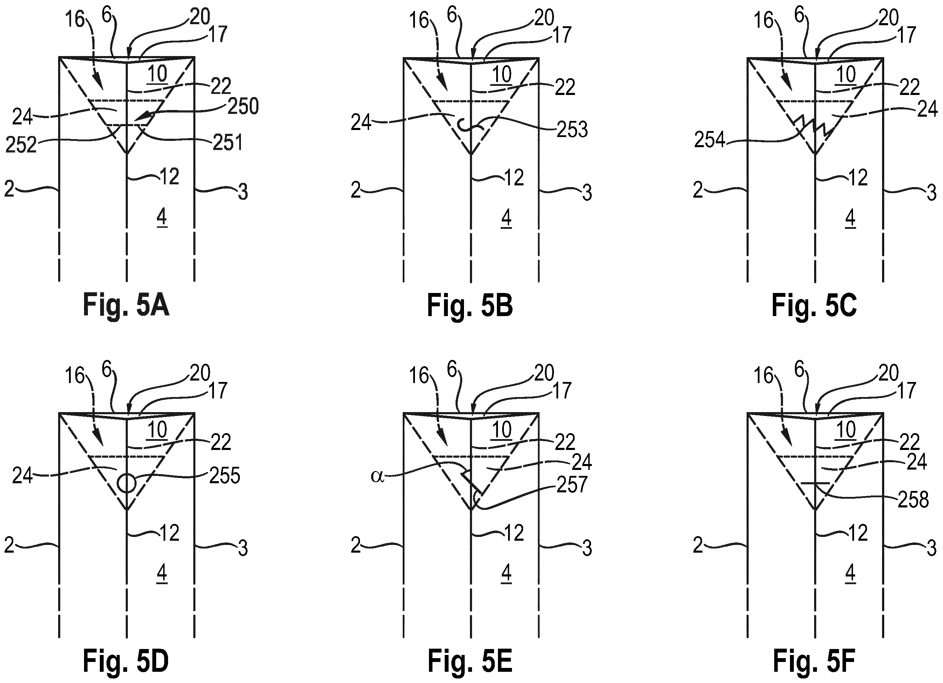

The area of weakness 25 may have a pattern selected from the group consisting of a straight line, a wavy line, a zigzag, a circle and combinations thereof, as exemplary shown in Fig. 5A-5I.

However, the area of weakness 25 is not a continuous slit. Otherwise, an opening may be formed and the compressed articles contained in the flexible package 1 may be in contact with the surrounding environment.

Generally, the flexible package 1 may be made of a film 110 having a thickness. The film 110 may have a thickness of less than 0.50 mm or less than 0.20 mm or less than 0.13 mm or less than 0.08 mm or less than 0.05 mm.

Figs. 5A to 5H show examples of where the weakened lines may be placed that are not covered by the present invention. As shown in Fig. 5A, the area of weakness 25 may comprise a plurality of straight lines 252 where the thickness of the film 110 is reduced or where the film 110 is perforated. The plurality of straight lines 252 alternates with a plurality of solid straight lines 251. Each straight line 252 has a length and each solid straight line 251 has a length. The ratio between the length of each straight line 252 and the length of each solid straight line 251 may be from 1:6 to 1:2 or from 1:4 to 1:2 or from 1:4 to 1:3.

The area of weakness 25 is therefore either a straight line of weakness comprising a plurality of lines where the thickness of the film 110 has been reduced or a perforated straight line. In both cases, the area of weakness 25 allows relieving the tension exerted on each gusset 10, 11 without the formation of a continuous slit, i.e. opening. Hence, the area of weakness 25 can prevent contamination of the compressed articles from dust, lint or moisture at least during the shelf life of the compressed articles.

The area of weakness 25 may be a straight line of weakness 257 which intersects the altitude 22 of the internal panel 16 of each gusset (10, 11) and forms an angle α ranging from
1 degree to 179 degrees or from 5 degrees to 175 degrees or from 10 degrees to 170 degrees, see for example Fig. 5E.

As shown in Fig. 5F, the straight line of weakness 258 may be perpendicular to the altitude 22 of the internal panel 16 of each gusset 10, 11. The straight line of weakness 258 may be also centered with regard to the altitude 22 of the internal panel 16 in order to better distribute the tension forces exerted on each gusset 10, 11.

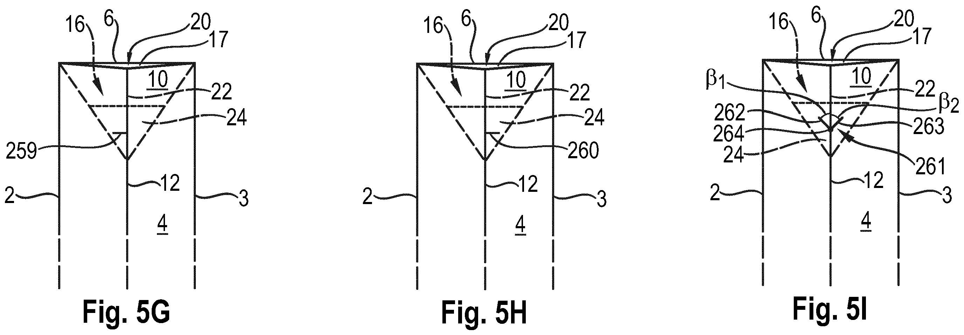

The area of weakness 25 may be a line of weakness 259, 260 which starts from the altitude 22 of the internal panel 16 of each gusset 10, 11 towards the front or rear wall 2, 3 (See for example Fig. 5G and 5H).

According to the present invention, the area of weakness 261 may comprise a first and second portion of weakness 262, 263 which converge at an end point 264. The first and second portion of weakness 262, 263 may be a straight line of weakness. The end point 264 of the area of weakness 261 may be located at or adjacent to the altitude 22 of the internal panel 16 of each gusset 10, 11. Each of the first and second portion of weakness 262, 263 may form a first and second angle β1, β2 with respect to the altitude 22 of the internal panel 16 of each gusset 10, 11. The first and second angle β1, β2 formed by the first and second portion of weakness 262, 263 with respect to the altitude 22 of the internal panel 16 of each gusset 10, 11 are angles adjacent to each other and are from 1 degree to 179 degrees or from 5 degrees to 175 degrees or from 10 degrees to 170 degrees, and from 2 degree to 358 degrees or from 10 degrees to 350 degrees or from 20 to 340 degrees together, see for example Fig. 5I.

The area of weakness 25 may selected from the group consisting of a straight line of weakness 257, 259, 260, a wavy line of weakness 253, a zigzag 254, a straight line of weakness 251 where the thickness of the film 110 is reduced or where the film 110 is perforated and combinations thereof. In those cases, the area of weakness 25 may have a length which is from 2 mm to 20 mm or from 2 mm to 10 mm or from 2 mm to 5 mm. The length of the area of weakness 25 should not be seen as the shortest distance from a starting point to an end point of the area of weakness 25 but as the completely length.

The area of weakness 25 may be a circle 255 having a diameter. The diameter of the circle 255 may be from 2 mm to 20 mm or from 2 mm to 10 mm or from 2 mm to 5 mm.

### The flexible package in a folded flat-shape

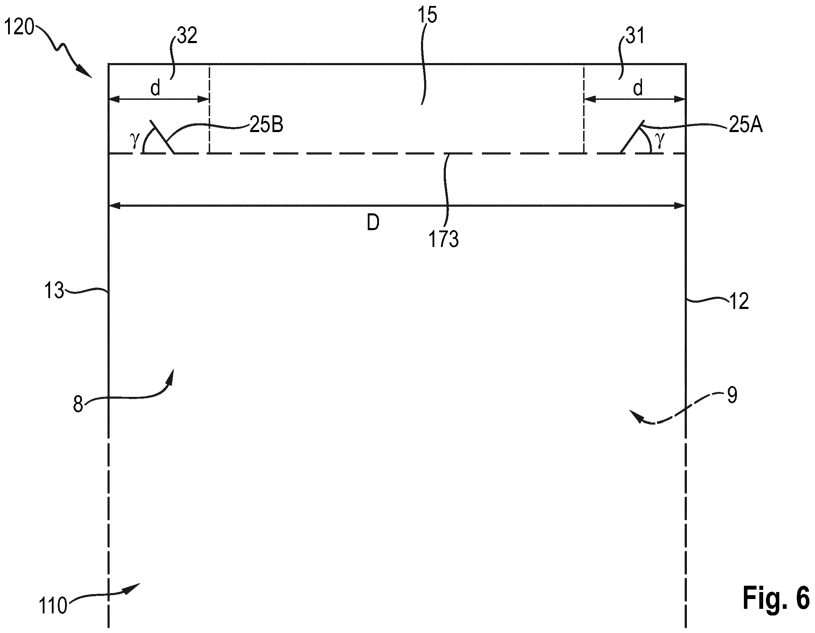

A flexible package 120 for accommodating a multiplicity of compressed articles is provided and has a substantially folded flat-shape. The flexible package 120 is able to be converted into the unfolded flexible package 1 having a substantially parallelepiped-shape. Fig. 6 shows a flexible package 120 within the scope of the invention.

When the flexible package 120 is converted into the unfolded flexible package 1, the flexible package 120 comprises a front wall 2, a rear wall 3, a first and second opposite side wall 4, 5, a third and fourth opposite side wall 6, 7 and a pair of gussets 10, 11 (See Fig. 1). When the flexible package 120 is converted into the unfolded flexible package 1, the flexible package 120 comprises a first panel 8 which forms the front wall 2, a portion of each of the first and second opposite side wall 4, 5 and a portion of the fourth side wall 7. When the flexible package 120 is converted into the unfolded flexible package 1, the flexible package 120 comprises a second panel 9 which forms the rear wall 3, a portion of each of the first and second opposite side wall 4, 5 and a portion of the fourth side wall 7.

As exemplary indicated in Fig. 6, the first and second panel 8, 9 are connected to each other along first and second side seams 12, 13 which are located at the first and second opposite side wall 4, 5 when the flexible package 120 is converted into the unfolded flexible package 1. The flexible package 120 comprises a third panel 15 which forms the third side wall 6 when the flexible package 120 is converted into the unfolded flexible package 1. The third panel 15 comprises the pair of gussets 10, 11. Each gusset 10, 11 is located at the respective first or second side wall 4, 5 when the flexible package 120 is converted into the unfolded flexible package 1.

When making the flexible package 120 from a film 110, the film 110 in the form of an endless sheet is folded over itself multiple times so as to form a generally M-shaped film. The film 110 is folded along a first, second and medial fold lines 171, 172, 173, as shown for instance in Fig. 2. The first, second and medial fold lines 171, 172, 173 extend in a machine direction. A central region of the film 110 is delimited by the two outer fold lines 171, 172.

The flexible package 120 comprises therefore a film 110 comprising a twofold portion 40 and a fourfold portion 50 which extends in the machine direction.

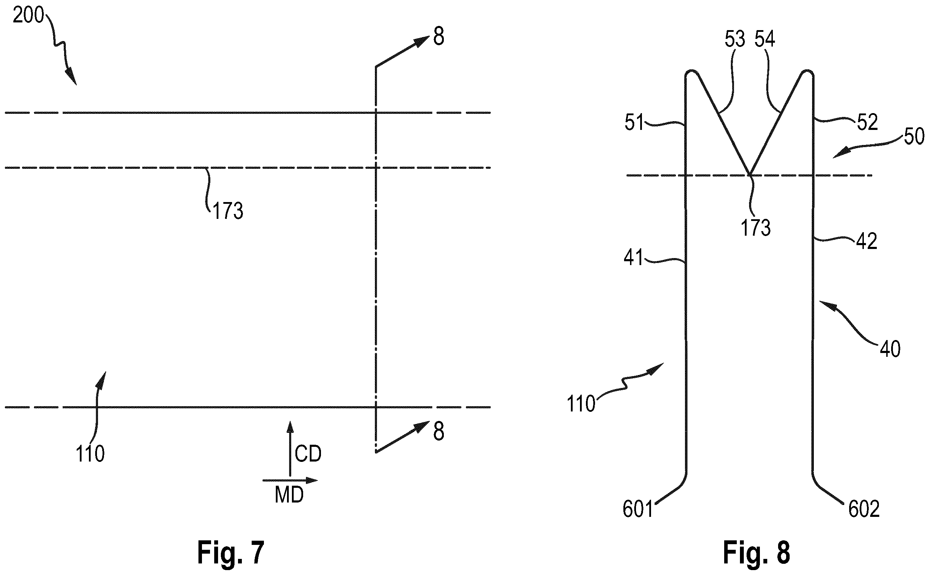

The twofold portion 40 of the film 110 comprises first and second layers 41, 42. The fourfold portion 50 of the film 110 comprises first and second outer layers 51, 52 and first and second inner layers 53, 54. The first and second inner layers 53, 54 are thus sandwiched between the first and second outer layers 51, 52 or the fourfold portion 50 of the film 110. The first and second outer layers 51, 52 of the fourfold portion 50 are coextensive with the first and second layers 41, 42 of the twofold portion 40 of the film 110 (see for example Fig. 8).

The first layer 41 of the twofold portion 40 of the film 110 forms the first panel 8 of the flexible package 120. The second layer 42 of the twofold portion of the film 110 forms the second panel 9 of the flexible package 120.

The first and second outer layers 51, 52 and the first and second inner layers 53, 54 of the fourfold portion 50 of the film 110 form the third panel 15. Hence, the third panel 15 is in a M-folded configuration, as shown in Fig. 6.

The first and second inner layers 53, 54 of the fourfold portion 50 of the film 110 mutually merge at the medial fold line 173. Each inner layer 53, 54 merges into the respective adjacent outer layer 51, 52 at the respective first and second fold lines 171, 172 of the film 110. The flexible package 120 thus comprises the medial fold line 173 in the third panel 15.

The medial fold line 173 of the third panel 15 has a length D from the first side seam 12 to the second side seam 13.

The third panel 15 comprises a first area 31 spaced inboard from the first side seam 12 to the second side seam 13. The third panel 15 comprises a second area 32 spaced inboard from the second side seam 13 to the first side seam 12. Each of the first and second area 31, 32 of the third panel 15 has a length d which is from 1% to 25% or from 1% to 15% or from 1% to 10% of the length D of the medial fold line 173 of the third panel 15.

The flexible package 120 comprises an area of weakness 25 which is located in at least one of the first and second area 31, 32 of the third panel 15.

The flexible package 120 may comprise an area of weakness 25 which is located in each of the first and second area 31, 32 of the third panel 15.

The flexible package 120 when converted into the unfolded package 1 may comprise a plurality of compressed articles that can exert tension on each gusset 10, 11 at each first and second opposite side wall 4, 5. An area of weakness 25 which is located in each of the first and second area 31, 32 of the third panel 15 may allow relieving the tension at each gusset 10, 11 at the respective first and second opposite side wall 4, 5 when formed.

The flexible package 120 comprises an area of weakness 25 which is located in at least one of the first or second inner layers 51, 52 of the fourfold portion 50 of the film 110.

In that case, the resulted flexible package 1 when unfolded may comprise as an area of weakness a straight line of weakness which starts from the altitude 22 of the internal panel 16 of each gusset 10, 11 towards the front or rear wall 2, 3.

The flexible package 120 may comprise an area of weakness 25 which is located in the first and second inner layers 51, 52 of the fourfold portion 50 of the film 110.

The area of weakness is located at or adjacent the medial fold line 173 of the third panel 15.

The area of weakness may be a straight line of weakness 25A, 25B which intersects the medial fold line 173 of the third panel 15 and forms an angle γ ranging from 1 degree to 179 degree or from 10 degrees to 170 degrees or from 40 degrees to 120 degrees.

In that case, the resulted flexible package 1 when unfolded may comprise a straight line of weakness which comprise a first and second portion of weakness 262, 263 which converge at an end point 264. The first and second portion of weakness 262, 263 is a straight line of weakness. The end point 264 of the area of weakness 261 may be located at or adjacent to the altitude 22 of the internal panel 16 of each gusset 10, 11. Each of the first and second portion of weakness 262, 263 may form a first and second angle β1, β2 with respect to the altitude 22 of the internal panel 16 of each gusset 10, 11. The first and second angle β1, β2 formed by the first and second portion of weakness 262, 263 with respect to the altitude 22 of the internal panel 16 of each gusset 10, 11 are angles adjacent to each other and are from 1 degree to 179 degrees or from 5 degrees to 175 degrees or from 10 degrees to 170 degrees, and from 2 degree to 358 degrees or from 10 degrees to 350 degrees or from 20 to 340 degrees together.

The flexible package 120 may comprise a straight line of weakness which is substantially perpendicular to the medial fold line 173 of the third panel 15. In that case, the resulted flexible package 1 when unfolded may comprise a straight line of weakness which is substantially perpendicular to the altitude 22 of the internal panel 16 of each gusset 10, 11. Hence, the straight line of weakness can allow distributing the tension exerted on each gusset 10, 11 of the flexible package 1.

### Methods of manufacturing the flexible package

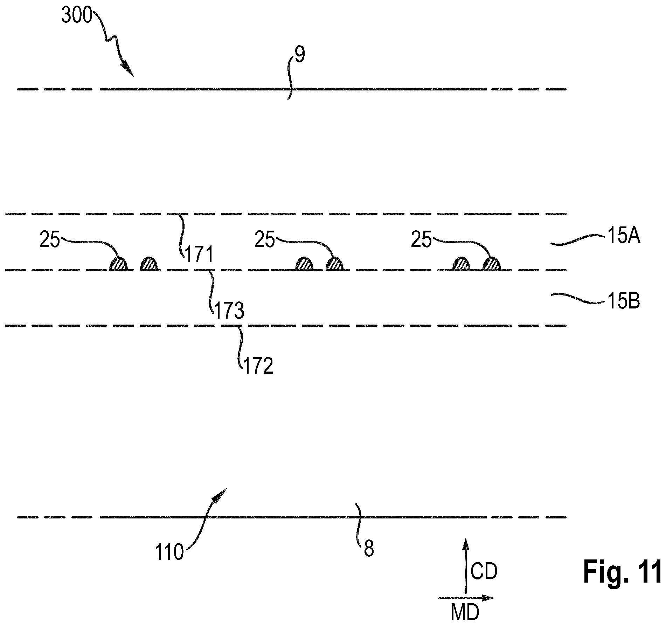

A method for producing flexible packages by automated machinery will now be briefly described. Fig. 7, 8, 9 and 10 schematically illustrate a first possible way of manufacturing the flexible package 120 for accommodating a multiplicity of compressed articles. Fig. 11 schematically illustrates a second possible way of manufacturing the flexible package 120 for accommodating a multiplicity of compressed articles The flexible package 120 has a substantially folded flat-shape which is able to converted into an unfolded flexible package 1 having a substantially parallelepiped-shape. The manufacture of the flexible package 120 may start from a film 110 in the form of an endless sheet travelling in a machine direction. The film 110 forms the package body. The film 110 may be constituted by any of the above described thermoplastic flexible films.

A method 200 of manufacturing the flexible package 120 is provided, the method comprising comprising a front wall 2, a rear wall 3, a first and second opposite side wall 4, 5, a third and fourth opposite side wall 6, 7 and a pair of gussets 10, 11 when the flexible package 120 is converted into the unfolded flexible package 1. The flexible package 120 comprises a first panel 8 which forms the front wall 2, a portion of each of the first and second opposite side wall 4, 5 and a portion of the fourth side wall 7 when the flexible package 120 is converted into the unfolded flexible package 1. The flexible package 120 comprises a second panel 9 which forms the rear wall 3, a portion of each of the first and second opposite side wall 4, 5 and a portion of the fourth side wall 7 when the flexible package 120 is converted into the unfolded flexible package 1.

The first and second panel 8, 9 are connected to each other along first and second side seams 12, 13 which are located at the first and second opposite side wall 4, 5 when the flexible package 120 is converted into the unfolded flexible package 1. The flexible package 120 comprises a third panel 15 which forms the third side wall 6 when the flexible package 120 is converted into the unfolded flexible package 1. The third panel 15 comprises the pair of gussets 10, 11. Each gusset 10, 11 is located at the respective first or second side wall 4, 5 when the flexible package 120 is converted into the unfolded flexible package 1.

### Folding of the film 110

The method 200 comprises the step of folding the film 110. When making the flexible package 120 from a film 110, the film 110 in the form of an endless sheet is folded over itself multiple times so as to form a generally M-shaped film. The film 110 is folded along a first, second and medial fold lines 171, 172, 173. The first, second and medial fold lines 171, 172, 173 extend in the machine direction. A central region of the film 110 is delimited by the two outer fold lines 171, 172.

The method 200 comprises the step of providing a film 110 in the form of an endless sheet travelling in a machine direction and comprising in a cross machine direction a twofold portion 40 and a fourfold portion 50 which extends in the machine direction, as shown in Fig. 7.

The twofold portion 40 of the film 110 comprises first and second layers 41, 42. The fourfold portion 50 of the film 110 comprises first and second outer layers 51, 52 and first and second inner layers 53, 54. The first and second inner layers 53, 54 are thus sandwiched between the first and second outer layers 51, 52 or the fourfold portion 50 of the film 110. The first and second outer layers 51, 52 of the fourfold portion 50 are coextensive with the first and second layers 41, 42 of the twofold portion 40 of the film 110 (see for example Fig. 8).

The first layer 41 of the twofold portion 40 of the film 110 forms the first panel 8 of the flexible package 120. The second layer 42 of the twofold portion of the film 110 from the second panel 9 of the flexible package 120.

The first and second outer layers 51, 52 and the first and second inner layers 53, 54 of the fourfold portion 50 of the film 110 form the third panel 15. Hence, the third panel 15 is in a M-folded configuration as shown in Fig. 6.

The first and second inner layers 53, 54 of the fourfold portion 50 of the film 110 mutually merge at the medial fold line 173. Each inner layer 53, 54 merges into the respective adjacent outer layer 51, 52 at the respective first and second fold lines 171, 172 of the film 110. The flexible package 120 thus comprises the medial fold line 173 in the third panel 15.

### Introducing areas of weakness

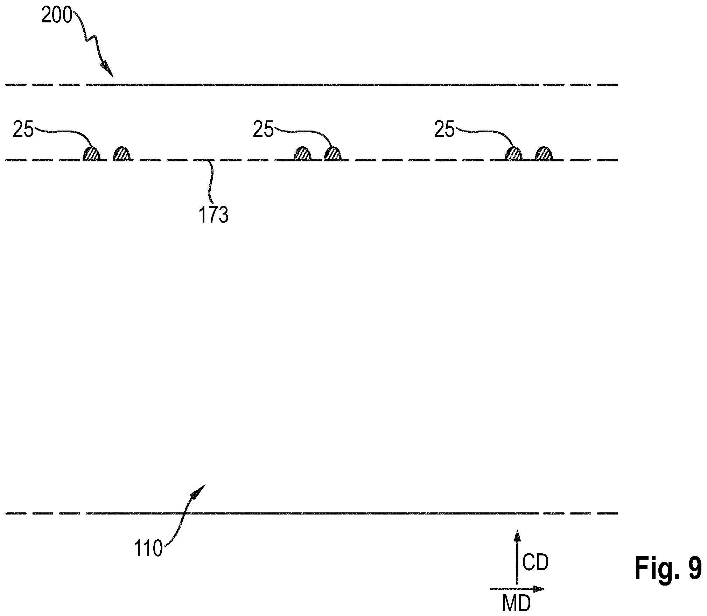

The method 200 comprises the step of introducing areas of weakness 25 at least at one of the first or second inner layer 53, 54 of the fourfold portion 50 of the film 110. The areas of weakness 25 are located at or adjacent the medial fold line 173 and in an area at or adjacent where the first and/or second side seams 12, 13 are formed, as exemplary shown in Fig. 9. The film 110 in a M-folded state may be opened by an appropriate device in order to get access to one of the first or second inner layer 53, 54 of the fourfold portion 50 of the film 110.

The method 200 may comprise the step of introducing areas of weakness 25 at the first and second inner layer 53, 54 of the fourfold portion 50 of the film 110. The areas of weakness 25 are located at or adjacent the medial fold line 173 and in an area at or adjacent where the first and/or second side seams 12, 13 are formed.

The areas of weakness 25 may be introduced by perforation with a knife or by using a laser beam. When using a laser beam, a focused spot of energy allows removing some film material at specific locations to a specified depth. Hence, areas of weakness comprising a plurality of straight lines 252 where the thickness of the film 110 is reduced or where the film 110 is perforated with a plurality of solid straight lines 253 may be produced by using a laser beam. Each straight line 252 has a length and each solid straight line 253 has a length. The ratio between the length of each straight line 252 and the length of each solid straight line 253 may be from 1:6 to 1:2 or from 1:4 to 1:2 or from 1:4 to 1:3.

When areas of weakness 25 are introduced in one of the first or second inner layer 53, 54 of the fourfold portion 50 of the film 110, the respective unaltered first or second inner layer 53, 54 may be preserved by the use of a plate made of steel for instance, which is placed between the first and second inner layer 53, 54 of the fourfold portion 50. Then, the laser beam may only weaken one of the first or second inner layer 53, 54 of the fourfold portion 50 of the film 110.

### Final sealing and cutting

The first and second layers 41, 42 of the twofold portion 40 of the film 110 are sealed to the outer and inner layers 51, 52, 53, 54 of the fourfold portion 50 of the film 110. The first and second layers 41, 42 of the twofold portion 40 of the film 110 sealed to the outer and inner layers 51, 52, 53, 54 of the fourfold portion 50 of the film 110 are simultaneously cut in the area where they are sealed.

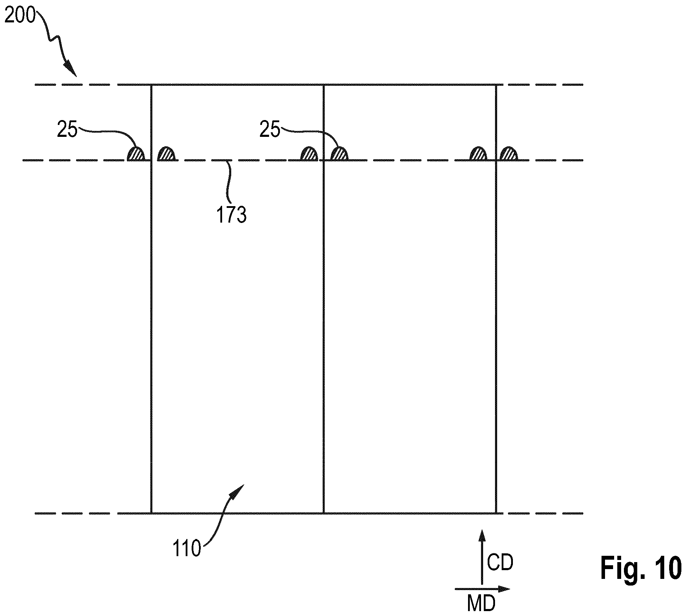

The sealing and cutting step is performed along a continuous straight line in the cross machine direction, which results in the formation of the two opposite side seams 12, 13 of the flexible package 120, as shown in Fig. 10.

An alternative method 300 of manufacturing the flexible package 120 is provided, the method comprising a front wall 2, a rear wall 3, a first and second opposite side wall 4, 5, a third and fourth opposite side wall 6, 7 and a pair of gussets 10, 11 when the flexible package 120 is converted into the unfolded flexible package 1. When the flexible package 120 is converted into the unfolded flexible package 1, the flexible package 120 comprises a first panel 8 which forms the front wall 2, a portion of each of the first and second opposite side wall 4, 5 and a portion of the fourth side wall 7. When the flexible package 120 is converted into the unfolded flexible package 1, the flexible package 120 comprises a second panel 9 which forms the rear wall 3, a portion of each of the first and second opposite side wall 4, 5 and a portion of the fourth side wall 7.

The first and second panel 8, 9 are connected to each other along first and second side seams 12, 13 which are located at the first and second opposite side wall 4, 5 when the flexible package 120 is converted into the unfolded flexible package 1. The flexible package 120 comprises a third panel 15 which forms the third side wall 6 when the flexible package 120 is converted into the unfolded flexible package 1. The third panel 15 comprises the pair of gussets 10, 11. Each gusset 10, 11 is located at the respective first or second side wall 4, 5 when the flexible package 120 is converted into the unfolded flexible package 1.

The method 300 comprises the step of providing a film 110 in the form of an endless sheet travelling in the machine direction. The film 110 comprises a first, second and medial fold lines 171, 172, 173 extending in the machine direction. A central region of the film 110 is delimited by the two outer fold lines 171, 172. The third panel 15 is thus delimited by the first and second fold line 171, 172. The medial fold line 173 is centered between the first and second fold line 171, 172 in the machine direction and divides the third panel 15 in a first and second half 15A, 15B in the machine direction, as shown in Fig. 11. When the film 110 is later M-folded, the first and second half 15A, 15B becomes the respective first and second inner layer 53, 54 of the fourfold portion 50 of the film 110.

### Introducing areas of weakness

The method 300 comprises the step of introducing areas of weakness 25 at least at one of the first or second half 15A, 15B of the third panel 15. The areas of weakness 25 are located at or adjacent the medial fold line 173 and in an area at or adjacent where the first and/or second side seams 12, 13 are formed, as exemplary shown in Fig. 11.

The method 300 may comprise the step of introducing areas of weakness 25 at the first and second half 15A, 15B of the third panel 15. The areas of weakness 25 are located at or adjacent the medial fold line 173 and in an area at or adjacent where the first and/or second side seams 12, 13 are formed, as exemplary shown in Fig. 11.

The areas of weakness 25 may be introduced by perforation with a knife or by using a laser beam as set out above.

In the method 300, the areas of weakness 25 are introduced in the film 110 in a flat state before any M-folding. Hence, the method 300 is a continuous method compared to the method 200. Also, the positioning of the areas of weakness 25 at one of the first or second half 15A, 15B of the third panel 15 which become one of the first or second inner layer 53, 54 of the fourfold portion 50 of the M-folded film 110 is less complex. The film 110 in the flat state does not require the use of any plate to protect one the first or second inner layer 53, 54. Any pattern for the area of weakness as set out above is therefore obtained conveniently.

In particular, the alternative method 300 is adequate in order to introduce a straight line of weakness 257 which intersects the altitude 22 of the internal panel 16 of each gusset 10, 11 and forms an angle α ranging from 1 degree to 179 degrees with respect with the altitude 22 of the internal panel 16 of each gusset 10, 11 in the flexible package 1. For this, a straight line of weakness is introduced at the first and second half 15A, 15B of the third panel 15. The areas of weakness 25 are located at or adjacent the medial fold line 173 and in an area at or adjacent where the first and/or second side seams 12, 13 are formed. The straight line of weakness forms an angle α ranging from 1 degree to 179 degrees or from 5 degrees to 175 degrees or from 10 degrees to 170 degrees with respect with the medial fold line 173 of the film 110.

### Folding of the film 110

The method 300 comprises the step of folding the film 110 in the cross machine direction to provide the film 110 comprising a twofold portion 40 and a fourfold portion 50.

The twofold portion 40 of the film 110 comprises first and second layers 41, 42. The fourfold portion 50 of the film 110 comprises first and second outer layers 51, 52 and first and second inner layers 53, 54. The first and second outer layers 51, 52 of the fourfold portion 50 are coextensive with the first and second layers 41, 42 of the twofold portion 40 of the film 110.

The first layer 41 of the twofold portion 40 of the film 110 forms the first panel 8 of the flexible package 120. The second layer 42 of the twofold portion of the film 110 from the second panel 9 of the flexible package 120.

The first and second outer layers 51, 52 and the first and second inner layers 53, 54 of the fourfold portion 50 of the film 110 form the third panel 15. Hence, the third panel 15 is in a M-folded configuration.

The first and second inner layers 53, 54 of the fourfold portion 50 of the film 110 mutually merge at the medial fold line 173.

### Final sealing and cutting

The method 300 comprises the step of sealing the first and second layers 41, 42 of the twofold portion 40 of the film 110 and the outer and inner layers 51, 52, 53, 54 of the fourfold portion 50 of the film 110. The first and second layers 41, 42 of the twofold portion 40 of the film 110 sealed to the outer and inner layers 51, 52, 53, 54 of the fourfold portion 50 of the film 110 are simultaneously cut in the area where they are sealed.

The sealing and cutting step is performed along a continuous straight line in the cross machine direction, which results in the formation of the two opposite side seams 12, 13 of the flexible package 120.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A flexible package (1) for accommodating a multiplicity of compressed articles, having a substantially parallelepiped-shape comprising:
a front wall (2), a rear wall (3), a first and second opposite side wall (4, 5), a third and fourth opposite side wall (6, 7) and a pair of gussets (10, 11);
a first panel (8), the first panel (8) forming the front wall (2), a portion of each of the first and second opposite side wall (4, 5) and a portion of the fourth side wall (7);
a second panel (9), the second panel (9) forming the rear wall (3), a portion of each of the first and second opposite side wall (4, 5) and a portion of the fourth side wall (7);
wherein the first and the second panel (8, 9) are connected to each other at the first and second opposite side wall (4, 5) along first and second side seams (12, 13);
a third panel (15), the third panel (15) forming the third side wall (6);
wherein the third panel (15) comprises the pair of gussets (10, 11);
wherein each gusset (10, 11) is located at the respective first or second side wall (4, 5);
wherein each gusset (10) comprises an internal panel (16) and an external panel (17);
wherein the internal panel and external panel (16, 17) of each gusset (10) are made of triangular portions of the third panel (15);
wherein the internal and external panel (16, 17) of each gusset (10) are coextensive at a first and second fold line (18, 19) to form a pocket (20);
wherein the first and second fold line (18, 19) of the pocket (20) of each gusset (10) converge and meet at a vertex (21);
wherein the vertex (21) of each gusset (10) is joined to the respective first or second side seam (12, 13) at the first or second side wall (4, 5);
wherein the internal panel (16) of each gusset (10) comprises an altitude (22) extending from the vertex (21) of each gusset (10) towards the third side wall (6);
wherein the altitude (22) of the internal panel (16) of each gusset (10) has a length (L);
wherein the internal panel (16) of each gusset (10) comprises a triangular area (24) which starts from the vertex (21) of each gusset (10) towards the third side wall (6);
wherein the triangular area (24) of the internal panel (16) of each gusset (10) has an altitude (27) which coincides with the altitude (22) of the internal panel (16) of each gusset (10);
wherein the altitude (27) of the triangular area (24) of the internal panel (16) of each gusset (10) has a length (1) which is from 1% to 30% of the length (L) of the altitude (22) of the internal panel (16) of each gusset (10);
wherein at least one gusset (10) comprises an area of weakness (25) located at least in the internal panel (16) of the at least one gusset (10) within the triangular area (24) of the internal panel (16) of the at least one gusset (10); and
wherein the area of weakness (25) is located at or adjacent to the altitude (22) of the internal panel (16) of the at least one gusset (10);
**characterized in that** the area of weakness (25) is not a continuous slit; and
wherein the area of weakness (261) comprises a first and second portion of weakness (262, 263) which converge at an end point (264), wherein the end point (264) is located at or adjacent to the altitude (22) of the internal panel (16) of each gusset (10, 11), wherein each of the first and second portion of weakness (262, 263) forms a first and second angle (β1, β2) with respect to the altitude (22) of the internal panel (16) of each gusset (10, 11), wherein the first and second angle (β1, β2) formed by the first and second portion of weakness (262, 263) with respect to the altitude (22) of the internal panel (16) of each gusset (10,11) are angles adjacent to each other and are from 1 degree to 179 degrees and are from 2 to 358 degrees together

2. The flexible package (1) of claim 1 wherein each gusset (10, 11) comprises an area of weakness (25) located at least within the triangular area (24) of the internal panel (16) of each gusset (10, 11),
wherein the area of weakness (25) is located at or adjacent to the altitude (22) of the internal panel (16) of each gusset (10, 11).

3. The flexible package (1) according to any of the preceding claims wherein the area of weakness (25) has a pattern selected from the group consisting of a straight line (250), a wavy line (253), a zigzag (254), a circle (255) and combinations thereof.

4. The flexible package (1) according to any of the preceding claims wherein the flexible package (1) is made of a film (110) having a thickness, wherein the area of weakness (250) comprises a plurality of straight lines (252) where the thickness of the film (110) is reduced or where the film (110) is perforated, wherein the plurality of straight lines (252) alternates with a plurality of solid straight lines (251), wherein each straight line (252) has a length and each solid straight line (251) has a length, wherein the ratio between the length of each straight line (252) and the length of each solid straight line (251) is from 1:6 to 1:2.

5. The flexible package (1) of any of the claims 1 or 4 wherein the area of weakness (25) is a straight line of weakness (257) which intersects the altitude (22) of the internal panel (16) of each gusset (10, 11) and forms an angle (α) ranging from 1 degree to 179 degrees with respect with the altitude (22) of the internal panel (16) of each gusset (10, 11).

6. The flexible package (1) of claim 5 wherein the area of weakness (25) is a straight line of weakness (259, 260) starts from the altitude (22) of the internal panel (16) of each gusset (10, 11) towards the front or rear wall (2, 3).

7. The flexible package (1) of any of the claims 3-6 wherein the area of weakness (25) is selected from the group consisting of a straight line of weakness (257, 259, 260), a wavy line of weakness (253), a zigzag (254), a straight line of weakness (251) where the thickness of the film (110) is reduced or where the film (110) is perforated and combinations thereof and wherein the area of weakness (25) has a length which is from 2 mm to 20 mm or wherein the area of weakness (25) is a circle (255) having a diameter, wherein the diameter of the circle (255) is from 2 mm to 20 mm.

## Patentansprüche

1. Elastische Verpackung (1) zum Aufnehmen einer Vielzahl komprimierter Artikel mit im Wesentlichen Parallelepipedform, umfassend:
eine Vorderwand (2), eine Rückwand (3), eine erste und zweite gegenüberliegende Seitenwand (4, 5), eine dritte und vierte gegenüberliegende Seitenwand (6, 7) und ein Verstärkungspaar (10, 11);
eine erstes Element (8), wobei das erste Element (8) die Vorderwand (2), einen Abschnitt jeder der ersten und zweiten gegenüberliegenden Seitenwand (4, 5) und einen Abschnitt der vierten Seitenwand (7) bildet;
ein zweites Element (9), wobei das zweite Element (9) die Rückwand (3), einen Abschnitt jeder der ersten und zweiten gegenüberliegenden Seitenwand (4, 5) und einen Abschnitt der vierten Seitenwand (7) bildet;
wobei das erste und das zweite Element (8, 9) an der ersten und zweiten gegenüberliegenden Seitenwand (4, 5) entlang erster und zweiter Seitennähte (12, 13) miteinander verbunden sind;
ein drittes Element (15), wobei das dritte Element (15) die dritte Seitenwand (6) bildet;
wobei das dritte Element (15) das Verstärkungspaar (10, 11) umfasst;
wobei sich jede Verstärkung (10, 11) an der ersten bzw. zweiten Seitenwand (4, 5) befindet;
wobei jede Verstärkung (10) ein Innenelement (16) und ein Außenelement (17) umfasst;
wobei das Innenelement und das Außenelement (16, 17) jeder Verstärkung (10) aus dreieckigen Abschnitten des dritten Elements (15) bestehen;
wobei das Innen- und Außenelement (16, 17) jeder Verstärkung (10) an einer ersten und zweiten Faltlinie (18, 19) coextensiv verlaufen, um eine Tasche (20) zu bilden;
wobei die erste und zweite Faltlinie (18, 19) der Tasche (20) jeder Verstärkung (10) zusammenlaufen und sich an einem Eckpunkt (21) treffen;
wobei der Eckpunkt (21) jeder Verstärkung (10) mit der ersten bzw. zweiten Seitennaht (12, 13) an der ersten oder zweiten Seitenwand (4, 5) verbunden ist;
wobei das Innenelement (16) jeder Verstärkung (10) eine Höhe (22) umfasst, die sich vom Eckpunkt (21) jeder Verstärkung (10) zur dritten Seitenwand (6) erstreckt;
wobei die Höhe (22) des Innenelements (16) jeder Verstärkung (10) eine Länge (L) aufweist;
wobei das Innenelement (16) jeder Verstärkung (10) eine dreieckige Fläche (24) umfasst, die am Eckpunkt (21) jeder Verstärkung (10) in Richtung der dritten Seitenwand (6) beginnt;
wobei die dreieckige Fläche (24) des Innenelements (16) jeder Verstärkung (10) eine Höhe (27) aufweist, die mit der Höhe (22) des Innenelements (16) jeder Verstärkung (10) übereinstimmt;
wobei die Höhe (27) der dreieckigen Fläche (24) des Innenelements (16) jeder Verstärkung (10) eine Länge (I) aufweist, die von 1 % bis 30 % der Länge (L) der Höhe (22) des Innenelements (16) jeder Verstärkung (10) beträgt;
wobei mindestens eine Verstärkung (10) einen Schwächungsbereich (25) aufweist, der sich mindestens im Innenelement (16) der mindestens einen Verstärkung (10) innerhalb der dreieckigen Fläche (24) des Innenelements (16) der mindestens einen Verstärkung (10) befindet; und
wobei sich der Schwächungsbereich (25) an der oder benachbart zur Höhe (22) des Innenelements (16) der mindestens einen Verstärkung (10) befindet;
**dadurch gekennzeichnet, dass** der Schwächungsbereich (25) kein durchgehender Schlitz ist; und
wobei der Schwächungsbereich (261) einen ersten und zweiten Schwächungsabschnitt (262, 263) umfasst, die an einem Endpunkt (264) zusammenlaufen, wobei sich der Endpunkt (264) an der oder benachbart zur Höhe (22) des Innenelements (16) jeder Verstärkung (10, 11) befindet, wobei jeder des ersten und zweiten Schwächungsabschnitts (262, 263) in Bezug auf die Höhe (22) des Innenelements (16) jeder Verstärkung (10, 11) einen ersten und zweiten Winkel (β1, β2) bildet, wobei der erste und zweite Winkel (β1, β2), die durch den ersten und zweiten Schwächungsabschnitt (262, 263) in Bezug auf die Höhe (22) des Innenelements (16) jeder Verstärkung (10, 11) gebildet werden, zueinander benachbarte Winkel sind und von 1 Grad bis 179 Grad betragen und zusammen von 2 Grad bis 358 Grad betragen.

2. Elastische Verpackung (1) nach Anspruch 1, wobei jede Verstärkung (10, 11) einen Schwächungsbereich (25) umfasst, der sich mindestens innerhalb der dreieckigen Fläche (24) des Innenelements (16) jeder Verstärkung (10, 11) befindet,
wobei sich der Schwächungsbereich (25) an der oder benachbart zur Höhe (22) des Innenelements (16) jeder Verstärkung (10, 11) befindet.

3. Elastische Verpackung (1) nach einem der vorstehenden Ansprüche, wobei der Schwächungsbereich (25) ein Muster aufweist, das ausgewählt ist aus der Gruppe, bestehend aus einer geraden Linie (250), einer Wellenlinie (253), einer Zickzacklinie (254), einem Kreis (255) und Kombinationen davon.

4. Elastische Verpackung (1) nach einem der vorstehenden Ansprüche, wobei die elastische Verpackung (1) aus einer Folie (110) mit einer Dicke besteht, wobei der Schwächungsbereich (250) eine Vielzahl gerader Linien (252) umfasst, bei denen die Dicke der Folie (110) verringert ist oder bei denen die Folie (110) perforiert ist, wobei sich die Vielzahl gerader Linien (252) mit einer Vielzahl durchgehender gerader Linien (251) abwechselt, wobei jede gerade Linie (252) eine Länge aufweist und jede durchgehende gerade Linie (251) eine Länge aufweist, wobei das Verhältnis zwischen der Länge jeder geraden Linie (252) und der Länge jeder durchgehenden geraden Linie (251) von 1:6 bis 1:2 beträgt.

5. Elastische Verpackung (1) nach einem der Ansprüche 1 oder 4, wobei der Schwächungsbereich (25) eine gerade Schwächungslinie (257) ist, die die Höhe (22) des Innenelements (16) jeder Verstärkung (10, 11) schneidet und in Bezug auf die Höhe (22) des Innenelements (16) jeder Verstärkung (10, 11) einen Winkel (α) bildet, der von 1 Grad bis 179 Grad reicht.

6. Elastische Verpackung (1) nach Anspruch 5, wobei der Schwächungsbereich (25) eine gerade Schwächungslinie (259, 260) ist, die an der Höhe (22) des Innenelements (16) jeder Verstärkung (10, 11) in Richtung der Vorder- oder Rückwand (2, 3) beginnt.

7. Elastische Verpackung (1) nach einem der Ansprüche 3 bis 6, wobei der Schwächungsbereich (25) ausgewählt ist aus der Gruppe, bestehend aus einer geraden Schwächungslinie (257, 259, 260), einer wellenförmigen Schwächungslinie (253), einer Zickzacklinie (254), einer geraden Schwächungslinie (251), bei dem die Dicke der Folie (110) verringert ist oder bei dem die Folie (110) perforiert ist und Kombinationen davon, und wobei der Schwächungsbereich (25) eine Länge aufweist, die von 2 mm bis 20 mm beträgt, oder wobei der Schwächungsbereich (25) ein Kreis (255) mit einem Durchmesser ist, wobei der Durchmesser des Kreises (255) von 2 mm bis 20 mm beträgt.

## Revendications

1. Conditionnement souple (1), destiné à contenir une multiplicité d'articles comprimés, de forme essentiellement parallélépipédique comprenant :
une paroi avant (2), une paroi arrière (3), une première et une deuxième parois latérales opposées (4, 5), une troisième et une quatrième parois latérales opposées (6, 7) et une paire de goussets (10, 11) ;
un premier panneau (8), le premier panneau (8) formant la paroi avant (2), une partie de chacune des première et deuxième parois latérales opposées (4, 5) et une partie de la quatrième paroi latérale (7) ;
un deuxième panneau (9), le deuxième panneau (9) formant la paroi arrière (3), une partie de chacune des première et deuxième parois latérales opposées (4, 5) et une partie de la quatrième paroi latérale (7) ;
dans lequel le premier et le deuxième panneaux (8, 9) sont raccordés l'un à l'autre au niveau des première et deuxième parois latérales opposées (4, 5) le long d'une première et d'une deuxième coutures latérales (12, 13) ;
un troisième panneau (15), le troisième panneau (15) formant la troisième paroi latérale (6) ;
dans lequel le troisième panneau (15) comprend la paire de goussets (10, 11);
dans lequel chaque gousset (10, 11) est situé respectivement au niveau de la première ou de la deuxième paroi latérale (4, 5) ;
dans lequel chaque gousset (10) comprend un panneau interne (16) et un panneau externe (17) ;
dans lequel les panneaux interne et externe (16, 17) de chaque gousset (10) sont faits de parties triangulaires du troisième panneau (15) ;
dans lequel les panneaux interne et externe (16, 17) de chaque gousset (10) sont coextensifs au niveau d'une première et d'une deuxième lignes de pliure (18, 19) afin de former une poche (20) ;
dans lequel les première et deuxième lignes de pliure (18, 19) de la poche (20) de chaque gousset (10) convergent et se réunissent en un sommet (21) ;
dans lequel le sommet (21) de chaque gousset (10) est respectivement attaché à la première ou à la deuxième couture latérale (12, 13) au niveau de la première ou de la deuxième paroi latérale (4, 5) ;
dans lequel le panneau interne (16) de chaque gousset (10) comprend une hauteur (22) s'étendant du sommet (21) de chaque gousset (10) vers la troisième paroi latérale (6) ;
dans lequel la hauteur (22) du panneau interne (16) de chaque gousset (10) a une longueur (L) ;
dans lequel le panneau interne (16) de chaque gousset (10) comprend une zone triangulaire (24) qui s'étend du sommet (21) de chaque gousset (10) vers la troisième paroi latérale (6) ;
dans lequel la zone triangulaire (24) du panneau interne (16) de chaque gousset (10) a une hauteur (27) qui coïncide avec la hauteur (22) du panneau interne (16) de chaque gousset (10) ;
dans lequel la hauteur (27) de la zone triangulaire (24) du panneau interne (16) de chaque gousset (10) a une longueur (I) qui va de 1 % à 30 % de la longueur (L) de la hauteur (22) du panneau interne (16) de chaque gousset (10) ;
dans lequel au moins un gousset (10) comprend une zone de faiblesse (25) située au moins dans le panneau interne (16) du au moins un gousset (10), à l'intérieur de la zone triangulaire (24) du panneau interne (16) du au moins un gousset (10) ; et
dans lequel la zone de faiblesse (25) est située au niveau de ou adjacente à la hauteur (22) du panneau interne (16) du au moins un gousset (10) ;
**caractérisé en ce que** la zone de faiblesse (25) n'est pas une fente continue ; et
dans lequel la zone de faiblesse (261) comprend une première et une deuxième parties de faiblesse (262, 263) qui convergent en un point d'extrémité (264), dans lequel le point d'extrémité (264) est situé au niveau de ou adjacent à la hauteur (22) du panneau interne (16) de chaque gousset (10, 11), dans lequel chacune des première et deuxième parties de faiblesse (262, 263) forme un premier et un deuxième angles (β1, β2) par rapport à la hauteur (22) du panneau interne (16) de chaque gousset (10, 11), dans lequel les premier et deuxième angles (β1, β2) formés par les première et deuxième parties de faiblesse (262, 263) avec la hauteur (22) du panneau interne (16) de chaque gousset (10, 11) sont des angles adjacents l'un par rapport à l'autre et mesurent de 1 degré à 179 degrés et, ensemble, de 2 à 358 degrés.

2. Conditionnement souple (1) selon la revendication 1, dans lequel chaque gousset (10, 11) comprend une zone de faiblesse (25) située au moins à l'intérieur de la zone triangulaire (24) du panneau interne (16) de chaque gousset (10, 11),
dans lequel la zone de faiblesse (25) est située au niveau de ou adjacente à la hauteur (22) du panneau interne (16) de chaque gousset (10, 11).

3. Conditionnement souple (1) selon l'une quelconque des revendications précédentes, dans lequel la zone de faiblesse (25) a un motif choisi dans le groupe constitué d'une ligne droite (250), d'une ligne ondulée (253), d'un zigzag (254), d'un cercle (255) et des combinaisons de ceux-ci.

4. Conditionnement souple (1) selon l'une quelconque des revendications précédentes, dans lequel le conditionnement souple (1) est constitué d'un film (110) ayant une épaisseur, dans lequel la zone de faiblesse (250) comprend une pluralité de lignes droites (252) où l'épaisseur du film (110) est réduite ou où le film (110) est perforé, dans lequel la pluralité de lignes droites (252) alterne avec une pluralité de lignes droites continues (251), dans lequel chaque ligne droite (252) a une longueur et chaque ligne droite continue (251) a une longueur, dans lequel le rapport entre la longueur de chaque ligne droite (252) et la longueur de chaque ligne droite continue (251) va de 1:6 à 1:2.

5. Conditionnement souple (1) selon l'une quelconque des revendications 1 ou 4, dans lequel la zone de faiblesse (25) est une ligne de faiblesse droite (257) qui croise la hauteur (22) du panneau interne (16) de chaque gousset (10, 11) et forme un angle (α) de 1 degré à 179 degrés par rapport à la hauteur (22) du panneau interne (16) de chaque gousset (10, 11).

6. Conditionnement souple (1) selon la revendication 5, dans lequel la zone de faiblesse (25) est une ligne de faiblesse droite (259, 260) qui s'étend de la hauteur (22) du panneau interne (16) de chaque gousset (10, 11) vers la paroi avant ou arrière (2, 3).

7. Conditionnement souple (1) selon l'une quelconque des revendications 3 à 6, dans lequel la zone de faiblesse (25) est choisie dans le groupe constitué d'une ligne de faiblesse droite (257, 259, 260), d'une ligne de faiblesse ondulée (253), d'un zigzag (254), d'une ligne de faiblesse droite (251) où l'épaisseur du film (110) est réduite ou où le film (110) est perforé et des combinaisons de ceux-ci et dans lequel la zone de faiblesse (25) a une longueur qui va de 2 mm à 20 mm ou dans lequel la zone de faiblesse (25) est un cercle (255) ayant un diamètre, dans lequel le diamètre du cercle (255) va de 2 mm à 20 mm.
